(19)

Europäisches Patentamt

European Patent Office

Office européen des brevets

(11) **EP 4 019 966 B1**

(12) **EUROPÄISCHE PATENTSCHRIFT**

(45) Veröffentlichungstag und Bekanntmachung des Hinweises auf die Patenterteilung:
**27.03.2024 Patentblatt 2024/13**

(21) Anmeldenummer: **21208410.7**

(22) Anmeldetag: **16.11.2021**

(51) Internationale Patentklassifikation (IPC):
**G01N 33/18** (2006.01)          **C02F 1/00** (2023.01)
**B01J 47/00** (2017.01)          **G01N 27/06** (2006.01)

(52) Gemeinsame Patentklassifikation (CPC):
**G01N 33/1846; B01J 47/14; C02F 1/42;**
**G01N 33/18;** C02F 2001/422; C02F 2001/425;
C02F 2209/005; C02F 2209/06; C02F 2209/20;
C02F 2303/16

(54) **VERFAHREN ZUM BESTIMMEN DER KIESELSÄURE-KONZENTRATION UND DER TOC-KONZENTRATION AM ENDE EINER VOLLENTSALZUNGSSTRASSE**

METHOD FOR DETERMINING SILICA AND TOC CONCENTRATION AT THE END OF A FULL DESALINATION LINE

PROCÉDÉ DE DÉTERMINATION DE LA CONCENTRATION D'ACIDE SILICIQUE ET DE COT À LA FIN D'UNE ROUTE DE DÉSALINISATION COMPLÈTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.12.2020 DE 102020134596**

(43) Veröffentlichungstag der Anmeldung:
**29.06.2022 Patentblatt 2022/26**

(73) Patentinhaber: **MionTec GmbH**
**51377 Leverkusen (DE)**

(72) Erfinder: **Mauer, Dieter**
**51377 Leverkusen (DE)**

(74) Vertreter: **Erbacher, Martin**
**Boehmert & Boehmert**
**Anwaltspartnerschaft mbB**
**Pettenkoferstrasse 22**
**80336 München (DE)**

(56) Entgegenhaltungen:
**DE-A1- 4 012 099          DE-U1-202010 007 452**
**US-A1- 2013 186 825**

• **WILHELM SEBASTIAN ET AL: "Influence of pH, Temperature and Sample Size on Natural and Enforced Syneresis of Precipitated Silica", POLYMERS, Bd. 7, Nr. 12, Dezember 2015 (2015-12), Seiten 2504-2521, XP055913264, DOI: 10.3390/polym7121528**

**Beschreibung**

Die vorliegende Erfindung betrifft ein Verfahren zum Bestimmen der Kieselsäure-Konzentration und der TOC-Konzentration am Ende einer Vollentsalzungsstraße.

HINTERGRUND DER ERFINDUNG

Mittels einer Vollentsalzungsstraße können durch Vollentsalzung sämtliche im Wasser gelösten Salze entfernt werden. Eine Vollentsalzungsstraße (VE-Straße) besteht immer aus einer Kationen- (KAT) und Anionenaustauscherstufe (AN). Aufgrund der durch einen Rieseler entfernten Anteile an $HCO_3^-$ ist der für den AN verbleibende anionische Anteil des Salzgehaltes immer kleiner als der kationische Anteil. Daher müssen VE-Straßen auf diese variablen Rohwassersituationen genau ausgelegt werden.

Das Problem bei der Berechnung der notwendigen Kapazitäten ist das unterschiedliche Alterungsverhalten der beiden Harzklassen. KATs verlieren ihre Kapazität fast nicht, während dies bei ANs der Fall ist. Daher wird die AN-Seite in der Praxis überdimensioniert und ist zu Beginn der Verwendung eines Ionenaustauschers kapazitätsmäßig größer als die KAT-Seite. In dieser Lebensphase erfolgt ein Durchbruch von Ionen in der VE-Straße am Beladeende über den sogenannten Na-Schlupf (das am wenigsten selektive Kation) durch den (kapazitätsmäßig kleineren) KAT. Nach ein paar Jahren sind beide Kapazitäten gleich und der Durchbruch kann sowohl über den KAT, als auch über den AN (über Kieselsäure, abgekürzt als $SiO_2$) erfolgen. Nach weiteren Betriebsjahren erfolgt er dann immer über die Kieselsäure. Dies ist jedoch problematisch, da $SiO_2$ im VE-Wasser unbedingt vermieden werden muss.

Ein weiteres Problem, das beim Betrieb einer Vollentsalzungsstraße auftritt, sind organische Moleküle im Kesselspeisewasser. Entsprechende organische Moleküle werden unter Kesselbedingungen weitgehend zu Säuren oxidiert, welche ein Korrosionspotential besitzen und daher auf ein Minimum reduziert werden sollten. Die Quelle dieser organischen Moleküle ist üblicherweise VE-Zusatzwasser, welches über die VE-Anlage aus dem Organik-haltigen Rohwasser erzeugt wird. Die Bindungsstärke (Selektivität) dieser organischen Anionen auf dem AN ist typischerweise nicht hoch, so dass beim Durchbruch der AN-Seite nicht nur $SiO_2$ auftritt, sondern auch Organik (gemessen als TOC; *total organic carbon*). Das Durchbruchsverhalten ist zwar vielfach erforscht worden, aber aufgrund der Vielgestalt organischer Moleküle nicht einheitlich. Trotzdem muss ein erhöhter Schlupf solcher organischer Säureanionen erkannt werden. Oft geschieht es, dass TOC bereits früh während des Beladelaufes und nicht erst am Beladeende erhöht im VE-Wasser erscheint.

Grundsätzlich ist ein Durchbruch des KAT gewünscht, da $Na^+$ leicht über den Leitfähigkeitsanstieg nach einer starkbasischen Anionenaustauscherstufe (SBA) detektiert werden kann und $Na^+$ als schlupfendes Ion unkritisch ist. Diese Messtechnik ist in jeder VE-Straße vorhanden und man versucht, die Auslegung mit so viel Kapazitätsreserve auf der AN-Seite auszustatten, dass dieser Wunschzustand möglichst lange erhalten bleibt.

Jedenfalls wird aber nach einer gewissen Zeit (die nur vom Grad der AN-Überdimensionierung abhängt), die Asymmetrierung doch in die falsche Richtung drehen und dann muss die Anlage dies anzeigen.

In der Praxis ergibt sich hinsichtlich der gedrehten Asymmetrie das Problem, dass die Kieselsäure (und auch andere unerwünschte schwache Säuren) durchzubrechen beginnen, ohne dass die Leitfähigkeit spürbar (über das Rauschen hinaus) ansteigt. Der pH-Wert fällt während dieser Zeit zwar ab, zeigt allerdings ebenfalls ein so starkes Rauschen, dass ein Rückschluss auf einen Kieselsäure und/oder TOC-Durchbruch auf dieser Grundlage in der Praxis mit gängigen und im akzeptablen Kostenrahmen liegenden pH-Messeinrichtungen nicht möglich ist.

Gemäß dem Stand der Technik werden dafür entweder teure online-Silikometer (photometrische Analysengeräte) eingesetzt oder diese Drehung der Asymmetrierung bleibt, in einfacheren Anlagen, unerkannt und führt dann zu zunehmendem $SiO_2$-Schlupf, welcher sehr kritisch für den Dampfturbinenbetrieb ist. Gleichermaßen steigt bei solchen Anlagen das Risiko des unerkannten erhöhten TOC-Schlupfes.

Bei größeren Anlagen mit erhöhtem Gefährdungspotential durch $SiO_2$-Durchbruch wird gemäß dem Stand der Technik ein online-Silikometer eingesetzt. Dieses wird dann meist aufgrund des hohen Preises von > 10 k€ mit einem Eingangsmultiplexer für mehrere Messpunkte gleichzeitig genutzt. Dies führt zu dem Problem, dass wichtige Kanäle mit schneller Änderungsgeschwindigkeit (nach SBA) gleichrangig mit langsam veränderlichen Messstellen (Kondensat, Speisewasser) verarbeitet werden. Somit werden oft Messintervalle von bis zu 1,5 Stunden erreicht. Das reicht für andere Messungen gut aus, ist für den VE-Straßen-AN aber in der Regel zu langsam. Eine Lösung wäre es, mehrere dieser teuren Geräte parallel zu verwenden, was allerdings zu einer signifikanten Kostenerhöhung führen würde.

Zur Bestimmung des Durchbruchs organischer Moleküle wird gemäß dem Stand der Technik in wenigen (eher großen) Anlagen ein online-TOC-Analysator eingesetzt, welcher typisch zwischen 15 und 30 k€ kostet. Auch hier ist die Analysenfrequenz ca. 15 min, welche durch die aufgrund des Gerätepreises verwendeten Eingangsmultiplexer noch um Faktoren von 2...6 erhöht wird.

Vollentsalzungsanlagen des Stands der Technik werden etwa in Mauer, Dieter: Vorgehensweisen gegen Chemikalienkostenanstieg in Vollentsalzungsanlagen bei Harzalterung. In: VGB PowerTech, 2012, No. 5, S. 80-84 beschrieben.

In der US 2013/0186825 A1 wird ein Verfahren zur Wasseraufbereitung beschrieben. Härte und Nicht-Hydroxid-Alkalität werden aus dem Speisewasser so weit entfernt, dass eine Verkalkung bei Aufkonzentrierung vermieden wird. Wenig ionisierbare Komponenten im Speisewasser werden durch Erhöhung des pH-Werts des Speisewassers zu einer verstärkten Ionisierung veranlasst. Auf diese Weise werden Stoffe wie Siliziumdioxid stark ionisiert, und (a) ihre Abscheidung durch die im Prozess verwendeten Membranen wird deutlich erhöht, und (b) ihre Löslichkeit im Rejektstrom des Membranprozesses wird deutlich erhöht. Wenig ionisierte Stoffe wie Bor, Kieselsäure und TOC werden in hohem Maße zurückgewiesen.

Wilhelm Sebastian et. Al., Polymers, 2015, 7(12) Seiten 2504-2521 untersucht den Einfluss von pH-Wert, Temperatur und Probengröße auf die natürliche und erzwungene Synärese von gefällter Kieselsäure.Es ist die der Erfindung zugrunde liegende Aufgabe, ein Verfahren für den Betrieb einer Vollentsalzungsstraße bereitzustellen, dass Nachteile des Stands der Technik überwindet, insbesondere ein Verfahren bereitzustellen, das es erlaubt, ein Drehen der Asymmetrierung entweder zuverlässig zu erkennen, um Gegenmaßnahmen einzuleiten, oder bei bereits gedrehter Asymmetrierung den Durchbruch nicht nur des Na, sondern auch der $SiO_2$ zuverlässig zu erkennen. Ebenso sollen erhöhte TOC-Werte erkannt werden, unabhängig davon wann diese innerhalb des Beladelaufes auftreten.

BESCHREIBUNG DER ERFINDUNG

Diese Aufgabe wird gemäß einem Verfahren nach Anspruch 1 gelöst. Weitere Ausführungsformen werden in den Unteransprüchen beschrieben.

Diese Aufgabe wird gelöst durch ein Verfahren zum Bestimmen der Kieselsäure-Konzentration und der TOC-Konzentration am Ende einer Vollentsalzungsstraße, umfassend die Schritte: a) Bereitstellen der Vollentsalzungsstraße, die zumindest ein Ionenaustauschersystem umfasst, wobei das Ionenaustauschersystem zumindest eine Kationenaustauscherstufe und zumindest eine Anionenaustauscherstufe umfasst; b) Betreiben der Vollentsalzungsstraße durch Durchleiten einer zu entsalzenden Lösung durch das Ionenaustauschersystem; c) Messen von n pH-Werten $pH_1$ bis $pH_n$ der Lösung nach der Anionenaustauscherstufe während des Betreibens der Vollentsalzungsstraße zu verschiedenen Zeitpunkten $t_1$ bis $t_n$; d) Berechnen eines Mittelwerts $pH_{gemittelt}$ aus den pH-Werten $pH_1$ bis $pH_n$; e) Messen eines aktuellen pH-Werts $pH_{n+1}$ zu einem Zeitpunkt $t_{n+1}$, wobei der Zeitpunkt $t_{n+1}$ zeitlich nach dem Zeitpunkt $t_n$ liegt; f) Berechnen einer pH-Differenz $\Delta pH$ zwischen dem Mittelwert $pH_{gemittelt}$ und dem aktuellen pH-Wert $pH_{n+1}$ nach $\Delta pH = pH_{gemittelt} - pH_{n-1}$; und g) Bestimmen der Kieselsäure-Konzentration nach der Anionenaustauscherstufe der Vollentsalzungsstraße und der TOC-Konzentration nach der Anionenaustauscherstufe der Vollentsalzungsstraße aus der pH-Differenz $\Delta pH$;

wobei

das Bestimmen in Schritt g) gemäß der folgenden Formel (II) erfolgt

$$c_{(SiO2+TOC)} = B + K \cdot \left(10^{\Delta pH} - 1\right) \qquad (II)$$

wobei $c_{(SiO2+TOC)}$ die gesamte Konzentration von Kieselsäure und TOC nach der Anionenaustauscherstufe in ppb ist; B von 0,01 bis 20 ppb ist; K von 30 bis 200 ppb ist;
und
das Verfahren ferner einen Schritt des Kalibrierens eines Messgeräts, das zum Messen der pH-Werte verwendet wird, umfasst, wobei das Kalibrieren in einem Zeitraum zwischen einem Zeitpunkt $t_0$ und dem Zeitpunkt $t_1$ erfolgt, wobei $t_0$ ein Zeitpunkt ist, der nach dem Beginn des Betreibens der Vollentsalzungsstraße liegt und ein Zeitraum zwischen dem Beginn des Betreibens der Vollentsalzungsstraße und $t_0$ etwa 5 % der Gesamtzeit des Betreibens der Vollentsalzungsstraße ist; und ein Zeitraum zwischen dem Beginn des Betreibens der Vollentsalzungsstraße und $t_1$ etwa 10 % der Gesamtzeit des Betreibens der Vollentsalzungsstraße ist.

Das erfindungsgemäße Verfahren hat große wirtschaftliche Vorteile, da es keine teuren online-Analysengeräte benötigt, sondern einfache, kostengünstige pH-Wert-Messgeräte zur quantitativen Ermittlung von $SiO_2$ und/oder organischen Säuren verwendet. Darüber hinaus ermöglicht das erfindungsgemäße Verfahren die quantitative Ermittlung von $SiO_2$ und/oder organischen Säuren mit einer überraschend hohen Messgenauigkeit.

Als Kieselsäuren werden die Sauerstoffsäuren des Siliziums bezeichnet. Die einfachste Kieselsäure ist Monokieselsäure (Orthokieselsäure) $Si(OH)_4$ oder auch $H_4SiO_4$. Sie ist eine schwache Säure ($pK_{S1}$ = 9,51; $pK_{S2}$ = 11,74) und neigt zur Kondensation. Wasserabspaltungen führen zu Verbindungen wie Dikieselsäure (Pyrokieselsäure) $(HO)_3Si-O-Si(OH)_3$ und Trikieselsäure $(HO)_3Si-O-Si(OH)_2-O-Si(OH)_3$. Cyclische (ringförmige) Kieselsäuren sind z. B. Cyclotrikieselsäure und Cyclotetrakieselsäure mit der allgemeinen Summenformel [Si(OH)$_2$-O-]n. Polymere werden gelegentlich als Metakieselsäure (H$_2$SiO$_3$, [-Si(OH)$_2$-O-]$_n$) bezeichnet. Kondensieren diese niedermolekularen Kieselsäuren weiter, bilden sich amorphe Kolloide (Kieselsol). Allgemeine Summenformel aller Kieselsäuren ist H$_{2n+2}$Si$_n$O$_{3n+1}$. Als Summenformel wird häufig SiO$_2$ · n H$_2$O angegeben; das Wasser ist bei Kieselsäuren jedoch kein Kristallwasser, sondern kann nur durch eine chemische Reaktion abgespalten werden und bildet sich aus konstitutionell gebundenen Hydroxygruppen. Erfindungsgemäß wird die Konzentration der Kieselsäure hinsichtlich einer Einheit SiO$_2$ angegeben.

Der gesamte organische Kohlenstoff (*total organic carbon,* TOC) ist ein Summenparameter und gibt die Summe des gesamten organischen Kohlenstoffs in einer Probe an. Er ist das Maß für den Gehalt an organischem Kohlenstoff in einer Probe. Der TOC schließt damit anorganisch gebundenen Kohlenstoff aus, wie zum Beispiel Carbonat, Hydrogencarbonat oder CO$_2$.

Eine Vollentsalzungsstraße dient dem Entfernen von Salzen aus Wasser, d. h. von Salzen, die in Wasser gelöst sind. Eine Vollentsalzungsstraße (VE-Straße) umfasst eine Kationen- (KAT) und Anionenaustauscherstufe (AN). Bei der Vollentsalzung werden sämtliche im Wasser gelösten Salze durch die Kombination von starksaurem Kationenaustauscher (evtl. unter Vorschaltung eines schwachsauren Kationenaustauschers) und starkbasischem Anionenaustauscher (evtl. unter Vorschaltung eines schwachbasischen Anionenaustauschers) entfernt. In der ersten Entsalzungsstufe erfolgt der Austausch aller Kationen gegen Wasserstoffionen. Die Konzentrationen der Anionen bleiben unverändert, d.h. es entstehen neben CO$_2$ in der Regel auch starke Säuren wie Salzsäure HCl, Salpetersäure HNO$_3$ und Schwefelsäure H$_2$SO$_4$. Dieser Vorgang wird deshalb auch als Entbasung bezeichnet. Die Regenerierung der Kationenaustauscher erfolgt vorzugsweise mit Salzsäure. Der saure Ablauf aus dem Kationenaustauscher wird in der zweiten Stufe über einen Anionenaustauscher geleitet. Wird ein schwachbasischer Austauscher eingesetzt, werden nur die Mineralsäuren entfernt (HCl, H$_2$SO$_4$, HNO$_3$). Wird dagegen ein starkbasischer Austauscher eingesetzt, kann auch Kieselsäure entzogen werden. Damit entsteht im Idealfall entmineralisiertes Wasser. Die Regeneration dieser Anionenaustauscher kann mit starken Basen wie z.B. Natronlauge erfolgen.

Ionentauscher oder Ionenaustauscher sind Materialien, mit denen gelöste Ionen durch andere Ionen gleichnamiger Ladung (d. h. Kationen oder Anionen) ersetzt werden können; es findet ein Ionentausch statt. Ionentauscher sind als Säulen, die mit einem Ionenaustauschermaterial gefüllt sind, oder als Membranen bekannt und werden von der zu behandelnden Lösung durchströmt. Die auszutauschenden Ionen werden am Ionenaustauschermaterial gebunden, das seinerseits dafür eine äquivalente Ladungsmenge von vorher gebundenen Ionen in die Lösung abgibt.

Je nachdem welche Art von Ionen ausgetauscht werden soll, unterscheidet man Kationenaustauscher und Anionenaustauscher. Im Fall eines Kationenaustauschers ist die aktive Gruppe eine anionische Gruppe, wie beispielsweise Sulfonsäure oder Carbonsäure, mit dissoziierbarem Kation. Man unterscheidet schwach saure Kationenaustauscher (etwa Carboxylgruppen) und stark saure (etwa Sulfonsäuregruppen).

Anionenaustauscher enthalten stark basische Gruppen, etwa quartäre Ammoniumgruppen als aktive Gruppen, die ihr Gegenion austauschen können. Generell werden Anionenaustauscher, die als Typ I und Typ II bezeichnet werden, verwendet. Die Unterscheidung der beiden Typen I und II bezieht sich auf die Eigenschaften für thermische Beständigkeit und Austauschkapazität. Ein Typ I-Anionenaustauscher ist thermisch stabiler, hat aber eine geringere Austauschkapazität als ein Typ II-Austauscher. Die austauschaktive Gruppe des Typ I-Austauschers ist beispielsweise eine Trialkylammonium-Gruppe (-N$^+$(CR$_3$)$_3$). Beim Typ II ist beispielsweise mindestens eine der drei Alkylgruppen durch eine Hydroxyalkylgruppe ersetzt.

Schwach basische Austauscher können Aminogruppen enthalten, an die die Anionen freier Säuren angelagert werden. Die Säureanionen sind reversibel gebunden. Mit Laugen werden bei der Regeneration die freien Aminogruppen wieder zurückgebildet. Abhängig vom Typ der Aminogruppe - primäre (-NH$_2$), sekundäre (-NHR) oder tertiäre Aminogruppe (-NR$_2$)- ist die Basizität der Austauscher leicht unterschiedlich.

Die zu entsalzende Lösung ist eine wässrige Lösung eines oder mehrerer Salze.

Der pH-Wert ist ein Maß für den sauren oder basischen Charakter einer wässrigen Lösung. Er ist der negative dekadische Logarithmus der Wasserstoffionenaktivität und eine dimensionslose Zahl. Der pH-Wert einer Lösung kann mit unterschiedlichen Methoden ermittelt werden, etwa durch Potentiometrie. Auf dem Prinzip der Potentiometrie beruhen die meisten handelsüblichen pH-Meter. Dabei wird eine mit Pufferlösung gefüllte Glasmembrankugel in die zu messende Flüssigkeit eingetaucht. Durch die Neigung der Wasserstoffionen, sich in dünner Schicht an Silikatgruppen der Glasoberfläche anzulagern, baut sich je nach pH-Differenz eine galvanische Spannung zwischen der Innen- und der Außenseite der Kugel auf. Diese elektromotorische Kraft wird mittels zweier Bezugselektroden gemessen, von denen sich eine innerhalb der Glaskugel und die andere in einem Referenzelektrolyten befindet.

Erfindungsgemäß werden n pH-Werte der Lösung, die die Anionenaustauscherstufe der Vollentsalzungsstraße verlässt, gemessen. Der erste pH-Wert (n=1) wird zum Zeitpunkt t$_1$ gemessen, der zweite pH-Wert (n=2) wird zum Zeitpunkt t$_2$ gemessen usw.

Typischerweise werden pH-Werte mit einem Aufzeichnungsintervall von ca. 1 sec. gemessen. Alle Berechnungen erfolgen dann ebenso in diesem Sekundentakt. Es kann vorgesehen sein, dass während des Verfahrens 100 bis 1000 pH-Werte aufgezeichnet werden, "n" also von 100 bis 1000 ist. Die pH-Werte werden während der gesamten Beladung der Vollentsalzungsstraße aufgezeichnet. Die Dauer der Beladung ist hierbei typischerweise zwischen 6 und 60 h. Eine Archivierung der Messwerte kann beispielsweise alle 20 bis 2.000 sec, je nach Beladedauer der Vollentsalzungsstraße und gewählter Punktanzahl, erfolgen.

Zu Beginn des Beladelaufes können während der ersten ca. 5 bis 10 % der gesamten Dauer der Beladung die Messwerte noch ignoriert werden, da die pH-Werte oft noch auf einen Gleichgewichtswert einschwingen.

Danach kann die Mittelwertbildung über die ab dann archivierten Messwerte starten. Diese Mittelwertbildung enthält dann eine fortwährend steigende Zahl der in der Mittelwertbildung enthaltenen Messwerte beginnend mit 1. Zur Mittelwertbildung werden vorzugsweise nur die archivierten Messwerte (vorzugsweise maximal weniger als 100 bis 1000) herangezogen.

Die Differenzbildung kann dann im Sekundentakt (bzw. der im Messgerät gewählten Messrate) zwischen dem aktuellen pH-Wert und dem alle ca. 20...2000 sec aktualisierten Mittelwert erfolgen. Die Erkennungszeit liegt also prinzipiell in der Größenordnung einer Sekunde, während das Rauschen von der in der Mittelwertbildung bereits eingerechneten Punktanzahl abhängt.

Das erfindungsgemäße Verfahren umfasst einen Schritt des Kalibrierens eines Messgeräts, das zum Messen der pH-Werte verwendet wird, umfasst, wobei das Kalibrieren in einem Zeitraum zwischen einem Zeitpunkt $t_0$ und dem Zeitpunkt $t_1$ erfolgt, wobei $t_0$ ein Zeitpunkt ist, der nach dem Beginn des Betreibens der Vollentsalzungsstraße liegt und ein Zeitraum zwischen dem Beginn des Betreibens der Vollentsalzungsstraße und $t_0$ etwa 5 % der Gesamtzeit des Betreibens der Vollentsalzungsstraße ist; und ein Zeitraum zwischen dem Beginn des Betreibens der Vollentsalzungsstraße und $t_1$ etwa 10 % der Gesamtzeit des Betreibens der Vollentsalzungsstraße ist.-In diesem Fall wird berücksichtigt, dass zum Beladebeginn fast nie ein $SiO_2$-Schlupf auftritt. Diese "Totzeit" über die ersten etwa 5-10 % der Zeit, bezogen auf die Dauer des gesamten Verfahrens, wird in dieser Ausführungsform genutzt, damit sich das Ionenaustauschersystem und die Messgeräte (insbesondere die Messgeräte, die für die Bestimmung der pH-Werte verwendet werden) in eine stabile Situation begeben können. Während dieses initialen Zeitraums kann die Messeinrichtung keine Aussage über $SiO_2$-Gehalte tätigen.

Zur Berechnung des Mittelwerts werden grundsätzlich alle gemessenen pH-Werte zwischen dem Beginn des Verfahrens ($t_1$) und dem zuletzt bestimmten pH-Wert berücksichtigt. Die Entwicklung dieses Mittelwertes zeigt ein immer weiter sinkendes Rauschen, je weiter die Beladung fortschreitet, da die Zahl der zur Berechnung benutzten Messwerte immer weiter zunimmt.

Das Berechnen des Mittelwerts $pH_{gemittelt}$ in Schritt d) kann gemäß der folgenden Formel (I) erfolgen

$$pH_{\text{gemittelt}} = \frac{pH_n}{n} + pH_{n-1,gemittelt} \cdot \left(1 - \frac{1}{n}\right) \qquad (I)$$

wobei n die Anzahl der nach $t_1$ gemessenen pH-Werte $pH_{gemittelt}$ zum Zeitpunkt der Bildung des Mittelwerts ist; und $pH_{n-1,gemittelt}$ der Mittelwert $pH_{gemittelt}$ zum Zeitpunkt $t_{n-1}$ ist.

Das Bestimmen in Schritt g) erfolgt gemäß der folgenden Formel (II)

$$c_{(SiO2+TOC)} = B + K \cdot \left(10^{\Delta pH} - 1\right) \qquad (II)$$

wobei $c_{(SiO2+TOC)}$ die gesamte Konzentration von Kieselsäure und TOC nach der Anionenaustauscherstufe in ppb ist; B von 0,01 bis 20 ppb, vorzugsweise von 1 bis 20 ppb, darüber hinaus bevorzugt von 4 bis 8 ppb ist; K von 30 bis 200 ppb, vorzugsweise 50 bis 100 ppb, am meisten bevorzugt 60 bis 70 ppb ist.

Die Konstante B ist ein Basiswert (Grundkonzentration an $SiO_2$ und TOC), der typischerweise zwischen 0,01 bis 20 ppb liegen kann (in der Praxis oft zwischen 3 bis 10 ppb) und bei $\Delta pH=0$, also früh während der Beladung, erhalten wird.

Die Konstante K wird empirisch ermittelt.

Darüber hinaus kann vorgesehen sein, dass das Ionenaustauschersystem eine zweiten Kationenaustauscherstufe umfasst; die zweite Kationenaustauscherstufe $Na^+$-Ionen aus der zu entsalzenden Lösung bindet und $H^+$-Ionen freisetzt; die zu entsalzende Lösung durch die zweiten Kationenaustauscherstufe geleitet wird nachdem die zu entsalzende Lösung durch die Anionenaustauscherstufe geleitet wurde; und die Schritte c) bis g) durchgeführt werden nachdem die zu entsalzenden Lösung durch die zweiten Kationenaustauscherstufe geleitet wurde. Hierdurch können störende und nichts aussagende Anteile im Wasser selektiv entfernt werden, um das Signal-zu-Rausch-Verhältnis der pH-Messung um einen Faktor 20 bis 40 zu verbessern.

Ebenso kann vorgesehen sein, dass das Messen des pH-Werts der Lösung nach der Anionenaustauscherstufe mittels einer Vorrichtung erfolgt, die sowohl Mittel zum Messen der Leitfähigkeit als auch Mittel zum Messen des pH-Werts umfasst.

Eine zusätzliche Messung der Leitfähigkeit, vorzugsweise ebenfalls nach der Anionenaustauscherstufe, kann durchgeführt werden, um die Verlässlichkeit der pH-Messung nach dem erfindungsgemäßen Verfahren zu kontrollieren und den Verbrauch der optional enthaltenden zweiten Kationenaustauscherstufe festzustellen. Nach einigen Monaten oder nach einer starken "Überfahrung" erreicht die Leitfähigkeit nach der zweiten Kationenaustauscherstufe regelmäßig nicht mehr Werte < 0,3 $\mu$S/cm. Dies zeigt dann, dass die Messung nicht mehr zuverlässig ist und die zweite Kationenaustauscherstufe durch eine frische Füllung ersetzt werden muss.

Es kann ferner vorgesehen sein, dass die Vorrichtung ferner weitere Mittel zum Messen der Leitfähigkeit und/oder Mittel zum Messen des pH-Werts umfasst, die es erlauben, die Leitfähigkeit und/oder den pH-Wert der zu entsalzenden Lösung in der Vollentsalzungsstraße vor dem Verlassen der Anionenaustauscherstufe zu bestimmen.

Ebenso kann hierbei vorgesehen sein, dass die Vorrichtung ferner eine oder mehrere Mittel umfasst, die aus der Gruppe ausgewählt sind, die aus einem Computer, einem Bildschirm und einer Software, die das gleichzeitige Durchführen des Verfahrens für zwei oder mehr Vollentsalzungsstraße erlaubt, besteht.

Eine entsprechende Vorrichtung, die in dem erfindungsgemäßen Verfahren zur Messung des pH-Werts und ggf. der Leitfähigkeit verwendet werden kann, ist das kommerziell erhältliche Mi-Vision-System.

DETAILIERTE BESCHREIBUNG DER ERFINDUNG

Im Folgenden soll die Erfindung unter Bezugnahme auf die beigefügten Figuren anhand konkreter Ausführungsbeispiele im Detail beschrieben werden. Die hinsichtlich der konkreten Beispiele genannten Merkmale sind hierbei nicht notwendigerweise wesentlich, um die Erfindung zu realisieren.

Fig. 1:    Mittelwert des pH und aktueller pH in Abhängigkeit vom Beladefortschritt;
Fig. 2:    Korrelation zwischen der Differenz aus aktuellem und gemitteltem (pH und $SiO_2$ + TOC)-Konzentration; und
Fig. 3:    Vergleich von Werten der $SiO_2$ Konzentration, die durch unterschiedliche Verfahren erhalten werden.

Das erfindungsgemäße Verfahren beruht in wesentlichen Teilen darauf, sehr kleine Abweichungen vom Normal-pH-Wert zu messen und zahlenmäßig zu interpretieren. Um eine ständige Notwendigkeit zur Nachkalibrierung dieser Messzelle zu vermeiden, wird erfindungsgemäß ein pH-Mittelwert unter stärkerer Betonung der Werte kurz nach Beginn des Beladelaufes gebildet. Die Mittelwertbildung erfolgt als gleitender Mittelwert mit einer linear steigenden Trägheit mit fortschreitendem Beladelauf. Wenn also der gemessene pH plötzlich zu fallen beginnt, wird der Mittelwert aufgrund seiner Trägheit erst einmal "geradeaus weiterlaufen", also deutlich langsamer absinken.

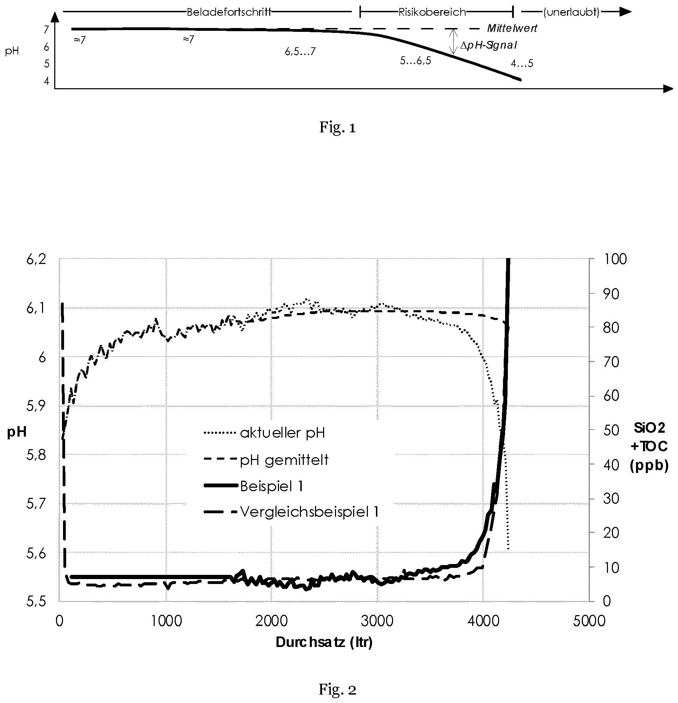

Dieses der Erfindung zugrundeliegende Prinzip wird in der Fig. 1 gezeigt.

Das $\Delta$pH-Signal wird als Differenz von Mittelwert (der vorangehenden Messungen) - (aktuellem) Messwert berechnet. Es wurde überraschenderweise durch die Erfinder festgestellt, dass das $\Delta$pH-Signal, potenziert und mit einer empirisch zu bestimmenden Konstante multipliziert, eine genaue Berechnung des $SiO_2$- und TOC-Gehalts ermöglicht.

*Beispiel 1 (Technikums-VE-Anlage)*

Gemäß dem erfindungsgemäßen Verfahren wurde im standardmäßigen Betrieb einer Vollentsalzungsstraße (mit einer Kationenaustauscherstufe bestehend aus schwachsauer Stufe (WAC) - starksauer Stufe (SAC), Rieseler sowie einer Anionenaustauscherstufe bestehend aus schwachbasischer Stufe (WBA) und starkbasischer Stufe (SBA)) mit dem Zweck, eine salzhaltige Lösung zu entsalzen, der pH-Wert der Lösung nach der Anionenaustauscherstufe wiederholt bestimmt.

Die Zulaufkonzentration war weitgehend konstant und betrug 2,25 meq/l Kationen, 0,81 meq/l Mineralsäureanionen (FMA) und 1,44 meq/l $HCO_3^-$. Die Restleitfähigkeit nach dieser VE-Straße lag mit ca. 0,3 $\mu$S/cm im guten, üblichen Bereich. Sie zeigte bis zum Durchbruch der Kieselsäure noch keinen messbaren Anstieg. Ein Beladeende über Na-Durchbruch durch den KAT trat also nicht auf und der Durchbruch über Kieselsäure wurde beobachtet.

Zur Messung des pH-Werts wurde ein kommerziell erhältliches Mi-Vision-Evaluierungssystem verwendet.

Die gemeinsame Konzentration an Kieselsäure und TOC wurde anschließend gemäß den oben angegebenen Formeln (I) und (II) bestimmt, wobei B (das heißt die Konzentration bei $\Delta$pH=0) 8 ppb war und K als empirisch bestimmter Wert von 65 ppb gewählt wurde.

*Vergleichsbeispiel 1 (handelsübliches analytisches Silikometer)*

Die Konzentration an Kieselsäure wurde gleichzeitig mit der Kurve in Beispiel 1 mittels eines aus dem Stand der Technik bekannten online-Silikometers gemessen. Diese (teure und daher nachteilhafte) Methode ermöglicht eine genaue Bestimmung der $SiO_2$-Konzentration und diente als Referenzmessung.

Die Ergebnisse der pH-Messungen des Beispiels 1 sowie die Ergebnisse der Bestimmung der gemeinsamen Konzentration an Kieselsäure und TOC nach Beispiel 1 und der Kieselsäurekonzentration nach Vergleichsbeispiel 1 werden in der Fig. 2 gezeigt.

*Beispiel 2 (Großanlage im Chemiestandort Hoechst)*

Das Beispiel 1 wurde an einer anderen VE-Anlage wiederholt. Es handelte sich um eine Straße bestehend aus den Stufen *SAC, Rieseler, WBA, SBA mit einem Produktionsfluss von ca. 200 m³/h. Die Zulauf Leitfähigkeit schwankte extrem stark zwischen ca. 50 und 800 μS/cm, da unregelmäßig Rückkehrkondensat zum Rohwasser beigemischt wurde. Diese Anlage stellte aufgrund der extremen Schwankungen im Zulauf besondere Anforderungen an die Kieselsäuremesstechnik. Auch in dieser Anlage konnte kein Na-Durchbruch durch den KAT gemessen werden, da die Harze in den AN Stufen schon sehr alt waren und daher der SiO$_2$-Durchbruch immer als erster auftrat. Dies sind ebenso wie im Beispiel 1 gute Voraussetzungen, den Kieselsäuredurchbruch detektieren zu können, bzw. sogar zu müssen.*

*Vergleichsbeispiel 2 (handelsübliches analytisches Silikometer)*

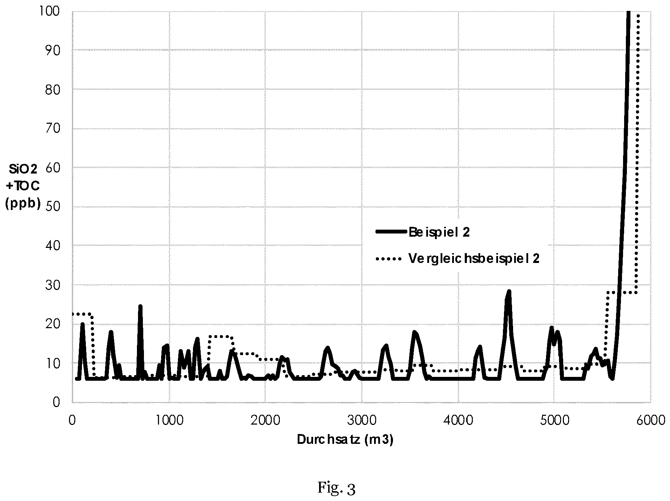

Als Vergleichsbeispiel 2 wurde wiederum eine zu Beispiel 2 zeitgleiche Messung mit einem online Silikometer durchgeführt, welches die Kieselsäurekonzentration als Referenzkurve zeigt. Die Ergebnisse der Bestimmung der gemeinsamen Konzentration an Kieselsäure und TOC nach Beispiel 2 und der Kieselsäurekonzentration in Vergleichsbeispiel 2 werden in der Fig. 3 gezeigt. In den Figuren 2 und 3 wird klar gezeigt, dass eine Bestimmung der Kieselsäure-Konzentration mittels des erfindungsgemäßen Verfahrens mit der Genauigkeit des deutlich teureren Referenzverfahrens (online-Silikometer) möglich ist. Messbare TOC-Werte traten in den hier aufgeführten Beispielen nicht auf.

**Patentansprüche**

1. Verfahren zum Bestimmen der Kieselsäure-Konzentration und der TOC-Konzentration am Ende einer Vollentsalzungsstraße, umfassend die Schritte:

   a) Bereitstellen der Vollentsalzungsstraße, die zumindest ein Ionenaustauschersystem umfasst, wobei das Ionenaustauschersystem zumindest eine Kationenaustauscherstufe und zumindest eine Anionenaustauscherstufe umfasst;
   b) Betreiben der Vollentsalzungsstraße durch Durchleiten einer zu entsalzenden Lösung durch das Ionenaustauschersystem;
   c) Messen von n pH-Werten $pH_1$ bis $pH_n$ der Lösung nach der Anionenaustauscherstufe während des Betreibens der Vollentsalzungsstraße zu verschiedenen Zeitpunkten $t_1$ bis $t_n$;
   d) Berechnen eines Mittelwerts $pH_{gemittelt}$ aus den pH-Werten $pH_1$ bis $pH_n$;
   e) Messen eines aktuellen pH-Werts $pH_{n+1}$ zu einem Zeitpunkt $t_{n+1}$, wobei der Zeitpunkt $t_{n+1}$ zeitlich nach dem Zeitpunkt $t_n$ liegt;
   f) Berechnen einer pH-Differenz $\Delta pH$ zwischen dem Mittelwert $pH_{gemittelt}$ und dem aktuellen pH-Wert $pH_{n+1}$ nach $\Delta pH = pH_{gemittelt} - pH_{n+1}$; und
   g) Bestimmen der Kieselsäure-Konzentration nach der Anionenaustauscherstufe der Vollentsalzungsstraße und der TOC-Konzentration nach der Anionenaustauscherstufe der Vollentsalzungsstraße aus der pH-Differenz $\Delta pH$;

   wobei

   das Bestimmen in Schritt g) gemäß der folgenden Formel (II) erfolgt

   $$c_{(SiO2+TOC)} = B + K \cdot \left(10^{\Delta pH} - 1\right) \qquad (II)$$

   wobei $c_{(SiO2+TOC)}$ die gesamte Konzentration von Kieselsäure und TOC nach der Anionenaustauscherstufe in ppb ist; B von 0,01 bis 20 ppb ist; K von 30 bis 200 ppb ist;
   und
   das Verfahren ferner einen Schritt des Kalibrierens eines Messgeräts, das zum Messen der pH-Werte verwendet wird, umfasst, wobei das Kalibrieren in einem Zeitraum zwischen einem Zeitpunkt $t_0$ und dem Zeitpunkt $t_1$ erfolgt, wobei $t_0$ ein Zeitpunkt ist, der nach dem Beginn des Betreibens der Vollentsalzungsstraße liegt und ein Zeitraum zwischen dem Beginn des Betreibens der Vollentsalzungsstraße und $t_0$ etwa 5 % der Gesamtzeit des Betreibens der Vollentsalzungsstraße ist; und ein Zeitraum zwischen dem Beginn des Betreibens der Vollentsalzungsstraße und $t_1$ etwa 10 % der Gesamtzeit des Betreibens der Vollentsalzungsstraße ist.

2. Verfahren nach Anspruch 1, wobei das Berechnen des Mittelwerts $pH_{\text{gemittelt}}$ in Schritt d) gemäß der folgenden Formel (I) erfolgt

$$pH_{\text{gemittelt}} = \frac{pH_n}{n} + pH_{n-1,gemittelt} \cdot \left(1 - \frac{1}{n}\right) \qquad (I)$$

wobei n die Anzahl der gemessenen pH-Werte $pH_{\text{gemittelt}}$ zum Zeitpunkt der Bildung des Mittelwerts ist; und $pH_{n-1,gemittelt}$ der Mittelwert $pH_{\text{gemittelt}}$ zum Zeitpunkt $t_{n-1}$ ist.

3. Verfahren nach Anspruch 1 oder 2, wobei das Messen des pH-Werts der Lösung nach der Anionenaustauscherstufe mittels einer Vorrichtung erfolgt, die sowohl Mittel zum Messen einer Leitfähigkeit als auch Mittel zum Messen des pH-Werts umfasst.

4. Verfahren nach Anspruch 3, wobei die Vorrichtung ferner weitere Mittel zum Messen der Leitfähigkeit und/oder Mittel zum Messen des pH-Werts umfasst, die es erlauben, die Leitfähigkeit und/oder den pH-Wert der zu entsalzenden Lösung in der Vollentsalzungsstraße vor und/oder während und/oder nach dem Verlassen der Anionenaustauscherstufe zu bestimmen.

5. Verfahren nach einem der Ansprüche 3 oder 4, wobei die Vorrichtung ferner eine oder mehrere Mittel umfasst, ausgewählt aus der Gruppe, bestehend aus einem Computer, einem Bildschirm und einer Software, die das gleichzeitige Durchführen des Verfahrens für zwei oder mehr Vollentsalzungsstraße erlaubt.

6. Verfahren nach einem der vorangehenden Ansprüche, wobei

das Ionenaustauschersystem eine zweite Kationenaustauscherstufe umfasst;
die zweite Kationenaustauscherstufe $Na^+$-Ionen aus der zu entsalzenden Lösung bindet und $H^+$-Ionen freisetzt;
die zu entsalzende Lösung durch die zweiten Kationenaustauscherstufe geleitet wird, nachdem die zu entsalzende Lösung durch die Anionenaustauscherstufe geleitet wurde; und
die Schritte c) bis g) durchgeführt werden, nachdem die zu entsalzenden Lösung durch die zweite Kationenaustauscherstufe geleitet wurde.

**Claims**

1. A method for determining the silica concentration and the TOC concentration at the end of a full desalination line, comprising the steps of:

a) providing the full desalination line comprising at least one ion exchange system, wherein the ion exchange system comprises at least one cation exchange stage and at least one anion exchange stage;
b) operating the full desalination line by passing a solution to be desalinated through the ion exchange system;
c) measuring n pH values $pH_1$ to $pH_n$ of the solution after the anion exchange stage during the operation of the full desalination line at different times $t_1$ to $t_n$;
d) calculating a mean value $pH_{\text{averaged}}$ from the pH values $pH_1$ to $pH_n$;
e) measuring a current pH value $pH_{n+1}$ at a time $t_{n+1}$, wherein the time $t_{n+1}$ is after the time $t_n$;
f) calculating a pH difference $\Delta pH$ between the mean value $pH_{\text{averaged}}$ and the current pH value $pH_{n+1}$ after $\Delta pH = pH_{\text{averaged}} - pH_{n+1}$; and
g) determining the silica concentration after the anion exchange stage of the full desalination line and the TOC concentration after the anion exchange stage of the full desalination line from the pH difference $\Delta pH$;

wherein

the determining in step g) is according to the following formula (II)

$$c_{(SiO2+TOC)} = B + K \cdot \left(10^{\Delta pH} - 1\right) \qquad (II)$$

wherein $c_{(SiO2+TOC)}$ is the total concentration of silica and TOC after the anion exchange stage in ppb; B is from 0.01 to 20 ppb; K is from 30 to 200 ppb; and

the method further comprises a step of calibrating a meter used for measuring the pH values, wherein the calibrating is in a time period between a time $t_0$ and the time $t_1$, wherein $t_0$ is a time that is after the start of the operation of the full desalination line and a time period between the start of the operation of the full desalination line and $t_0$ is about 5% of the total time of the operation of the full desalination line; and a time period between the start of the operation of the full desalination line and $t_1$ is about 10% of the total time of the operation of the full desalination line.

2. The method according to claim 1, wherein the calculating the mean value pH averaged in step d) is according to the following formula (I)

$$pH_{\text{averaged}} = \frac{pH_n}{n} + pH_{n-1,averaged} \cdot \left(1 - \frac{1}{n}\right) \qquad (I)$$

wherein n is the number of measured pH values at the time of calculating the mean value $pH_{\text{averaged}}$; and $pH_{n-1,\ averaged}$ is the mean value $pH_{\text{averaged}}$ at the time $t_{n-1}$.

3. The method according to claim 1 or 2, wherein the measuring of the pH value of the solution after the anion exchange stage is performed by means of a device comprising both means for measuring a conductivity and means for measuring the pH value.

4. The method according to claim 3, wherein the device further comprises further means for measuring the conductivity and/or means for measuring the pH value allowing to determine the conductivity and/or the pH value of the solution to be desalinated in the full desalination line before and/or during and/or after leaving the anion exchange stage.

5. The method according to any one of claims 3 or 4, wherein the device further comprises one or more means selected from the group consisting of a computer, a screen and software allowing to perform the method simultaneously for two or more full desalination lines.

6. The method according to any one of the preceding claims, wherein

the ion exchange system comprises a second cation exchange stage;
the second cation exchange stage binds $Na^+$ ions from the solution to be desalinated and releases $H^+$ ions;
the solution to be desalinated is passed through the second cation exchange stage after the solution to be desalinated has been passed through the anion exchange stage; and
steps c) to g) are performed after the solution to be desalinated has been passed through the second cation exchange stage.

## Revendications

1. Procédé de détermination de la concentration en silice et de la concentration en carbone organique total (TOC) en sortie d'une ligne de déminéralisation totale, ledit procédé comprenant les étapes suivantes :

a) fournir la ligne de déminéralisation totale qui comprend au moins un système d'échange d'ions, le système d'échange d'ions comprenant au moins un étage d'échange de cations et au moins un étage d'échange d'anions ;
b) faire fonctionner la ligne de déminéralisation totale par passage d'une solution à déminéraliser à travers le système d'échange d'ions ;
c) mesurer n valeurs de pH $pH_1$ à $pH_n$ de la solution après l'étage d'échange d'anions pendant le fonctionnement de la ligne de déminéralisation à différents instants $t_1$ à $t_n$ ;
d) calculer une valeur moyenne $pH_{gemittelt}$ à partir des valeurs de pH $pH_1$ à $pH_n$ ;
e) mesurer une valeur de pH actuelle $pH_{n+1}$ à un instant $t_{n+1}$, l'instant $t_{n+1}$ étant postérieur à l'instant $t_n$ ;

f) calculer une différence de pH $\Delta$pH entre la valeur moyenne $pH_{gemittelt}$ et la valeur de pH actuelle $pH_{n+1}$, conformément à $\Delta pH = pH_{gemittelt} - pH_{n+1}$ ; et

g) déterminer la concentration en silice après l'étage d'échangeur d'anions de la ligne de déminéralisation totale et la concentration en TOC après l'étage d'échangeur d'anions de la ligne de déminéralisation totale à partir de la différence de pH $\Delta$pH ;

la détermination à l'étape g) étant effectuée selon la formule (II) suivante

$$C_{(SiO2+TOC)} = B + K \cdot (10^{\Delta pH} - 1) \quad (II)$$

$C_{(SiO2+TOC)}$ étant la concentration totale en silice et TOC après l'étage d'échange d'anions en ppb ; B étant compris entre 0,01 et 20 ppb ; K étant compris entre 30 et 200 ppb ;
et

le procédé comprenant en outre une étape d'étalonnage d'un appareil de mesure qui est utilisé pour mesurer les valeurs de pH, l'étalonnage s'effectuant dans un intervalle de temps compris entre un instant $t_0$ et l'instant $t_1$, $t_0$ étant un instant après le début du fonctionnement de la ligne de déminéralisation totale et un intervalle de temps entre le début du fonctionnement de la ligne de déminéralisation totale et $t_0$ étant d'environ 5 % de la durée totale de fonctionnement de la ligne de déminéralisation totale ; et un intervalle de temps entre le début du fonctionnement de la ligne de déminéralisation totale et $t_1$ représentant environ 10 % de la durée totale du fonctionnement de la ligne de déminéralisation totale.

2.  Procédé selon la revendication 1, **caractérisé en ce que** le calcul de la valeur moyenne $pH_{gemittelt}$ à l'étape d) est effectué selon la formule (I) suivante

$$pH_{gemittelt} = \frac{pH_n}{n} + pH_{n-1,gemittelt} \cdot \left(1 - \frac{1}{n}\right) \quad (I)$$

n étant le nombre de valeurs de pH mesurées $pH_{gemittelt}$ à l'instant où la valeur moyenne est formée ; et $pH_{n-1,gemittelt}$ étant la valeur moyenne $pH_{gemittelt}$ à l'instant $t_{n-1}$.

3.  Procédé selon la revendication 1 ou 2, la mesure de la valeur de pH de la solution étant effectuée après l'étage d'échange d'anions au moyen d'un dispositif qui comprend aussi bien des moyens de mesure de conductivité que des moyens de mesure de valeur de pH.

4.  Procédé selon la revendication 3, le dispositif comprenant en outre des moyens de mesure de conductivité et/ou des moyens de mesure de valeur de pH qui permettent de mesurer la conductivité et/ou la valeur de pH de la solution à déminéraliser dans la ligne de déminéralisation totale et/ou pendant et/ou après la sortie de l'étage d'échange d'anions.

5.  Procédé selon l'une des revendications 3 ou 4, le dispositif comprenant en outre un ou plusieurs moyens choisis dans le groupe comprenant un ordinateur, un écran et un logiciel qui permet de mettre en œuvre simultanément le procédé pour deux ou plusieurs lignes de déminéralisation totale.

6.  Procédé selon l'une des revendications précédentes,

le système d'échange d'ions comprenant un deuxième étage d'échange de cations ;
le deuxième étage d'échange de cations liant les ions Na$^+$ provenant de la solution à déminéraliser et libérant des ions H$^+$ ;
la solution à déminéraliser passant par le deuxième étage d'échange de cations après que la solution à déminéraliser est passée par l'étage d'échange d'anions ; et
les étapes c) à g) étant réalisées après passage de la solution à déminéraliser par le deuxième étage d'échange de cations.

Fig. 1

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20130186825 A1

**In der Beschreibung aufgeführte Nicht-Patentliteratur**

- **MAUER, DIETER.** Vorgehensweisen gegen Chemikalienkostenanstieg in Vollentsalzungsanlagen bei Harzalterung. *VGB PowerTech,* 2012, (5), 80-84

- **WILHELM SEBASTIAN.** *Polymers,* 2015, vol. 7 (12), 2504-2521